# EUROPEAN PATENT APPLICATION

(11) **EP 3 488 762 A1**
(43) Date of publication of application: **29.05.2019**
(21) Application number: 17831121.3
(22) Date of filing: 20.07.2017
(51) Int. Cl.: A61B 1/313, A61B 1/00, A61B 1/015

(54) **ENDOSCOPIC DEVICE AND ENDOSCOPIC SYSTEM**

(30) Priority: 22.07.2016 JP 2016144323
(71) Applicant: Osaka University, Suita-shi, Osaka 565-0871 (JP)
(72) Inventor: NAKAJIMA, Kiyokazu, Suita-shi Osaka 565-0871 (JP)
(74) Representative: Vigand, Philippe
(86) International application number: PCT/JP2017/026351
(87) International publication number: WO 2018/016605

(57) **Abstract**

Provided is: an endoscopic device used for constructing an endoscopic system, which is simpler in structure and lower in cost as compared with conventional devices; and an endoscopic system using same. A hard-type laparoscopic device 111 has an optical viewing tube 121 equipped with observation optical systems 123, 125a-125d and an illumination optical system 122, the device being characterized in that a storage part 131 which houses a light source 141 which provides illumination light to the illumination optical system 122 and a gas cylinder 151 for supplying gas to insufflate the abdominal cavity is disposed in the optical viewing tube 121 in such a manner as to allow the optical viewing tube 121, the light source 141, and the gas cylinder 151 to be held together as a rigid integral unit.

## Description

### Technical Field

The present invention relates to an endoscopic device for an endoscopic surgical operation and to an endoscopic system including the endoscopic device. More specifically, the present invention relates to a novel rigid laparoscopic device and a novel rigid laparoscopic system each for a surgical operation that requires securing of a visual field by pneumoperitoneum.

### Background Art

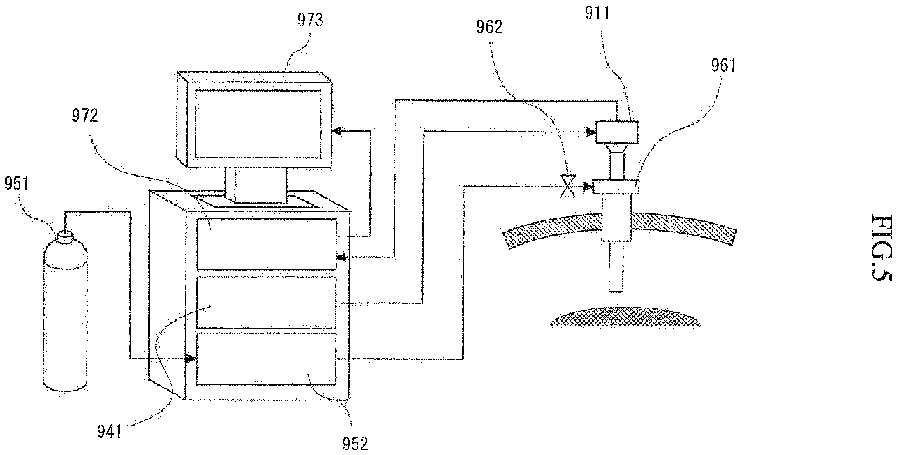

FIG. 5 illustrates a general system used to internally observe an abdominal cavity with an endoscope. Currently, for the internal observation of the abdominal cavity, a rigid endoscope is typically used to display an image of the inside of the body on a display device 973 and to see the image. During such an operation, it is necessary to secure a visual field and an operative field inside the body. For this purpose, a surgical technique involving use of pneumoperitoneum is widely employed. According to the pneumoperitoneum, carbon dioxide gas is supplied into the abdominal cavity so that the abdomen is swelled.

Specifically, as illustrated in FIG. 5, a small-diameter incision is first made on a surface of a patient's body. Through the incision, a trocar 961 is inserted into the body. Then, an optical viewing tube 911 is inserted into the body cavity through the trocar 961. The optical viewing tube 911 is provided with an observation optical system and an illumination optical system. An image of an observation object is obtained by the observation optical system, and the image is displayed on the display device 973 via a video processor 972.

The trocar 961 has a gas port 962 that is provided with a valve and is used for pneumoperitoneum. Through the gas port 962, gas having been subjected to flow rate regulation by a pneumoperitoneum device 952 is supplied into the abdominal cavity.

Thus, in order to perform a laparoscopic surgery, the optical viewing tube 911 and surgical instruments selected according to the surgery, such as forceps, are required. In addition to them, various other devices are required, such as the video processor 972, the display device 973, a light source device 941 for supplying light to the illumination optical system of the optical viewing tube, a gas cylinder 951 for supplying gas into the abdominal cavity, and the pneumoperitoneum device 952 for regulating a flow rate of the gas. Typically, these devices and instruments are collectively arranged in an operating room, so that these devices and instruments function as an integrated system.

Meanwhile, the idea of universal health coverage (UHC) are becoming popular in recent years. The "UHC" means that "all individuals can receive the services including appropriate health promotion, prevention, treatment, and rehabilitation at payable cost". The UHC aims to provide healthcare services to many people without financial hardship. This idea of UHC is promoted also in the field of the endoscopic surgical operation.

### Citation List

### Patent Literature

Patent Literature 1: JP 2014-113256 A

### Summary of Invention

### Technical Problem

The above devices and instruments are typically placed in a dedicated tower rack. However, still more space for the devices and instruments to be placed is required. In addition, each of these devices is premised to be used in an operating room for advanced medical care. Thus, efforts for downsizing and simplifying the entire device for the purpose of portability have not been made. Rather, excessively higher functionality has been pursued. This results in an increase in size, an increase in cost, and difficulty in use of the system.

Meanwhile, even under an environment that is not necessarily well-ordered enough to provide high medical care, such as in wide-scale disaster sites, there is a need for, e.g., medical examination and relatively simple treatment in the abdominal cavity with use of an endoscope. However, the above-described advanced, large-scale system is expensive, and is difficult to be used in the situations described above. In addition, in the situations described above, a high-performance system is not necessarily required. Rather, from the viewpoint of UHC, a simple, easy-to-use, and low-cost endoscopic system is required occasionally.

In view of the circumstances described above, an object of the present invention is to provide: an endoscopic device for constituting an endoscopic system that is smaller in size, lighter in weight, simpler in configuration, or lower in cost than the conventional systems and that does not require a higher skill or practice to use the system; and an endoscopic system including the endoscopic device. Solution to Problem

In order to attain the object, an endoscopic device according to the present invention is a rigid laparoscopic device including an optical viewing tube including an observation optical system and an illumination optical system, wherein an accommodation section in which a light source for supplying illumination light to the illumination optical system and a gas cylinder for supplying gas for pneumoperitoneum are accommodated is provided to the optical viewing tube to allow the optical viewing tube, the light source, and the gas cylinder to be held integrally and with rigidity.

In the rigid laparoscopic device described above, a small light source device and a small gas cylinder are employed so that the light source device, the gas cylinder, and the optical viewing tube can be held integrally and rigidly. This can save a space for the light source device and the gas cylinder. More specifically, since the light source device and the optical viewing tube are integrally configured, it is possible to omit a light guide cable for guiding light from the light source device to the optical viewing tube, for example. Particularly, according to the present invention, the above-described configuration is applied to the rigid laparoscope among various endoscopes. This enables the whole of the device to achieve a compact, integrated structure with higher rigidity, and also allows an operator to operate the laparoscope with his/her single hand.

In addition, the above-described configuration makes it easy for the operator to manually perform, at a location close to his/her hand, an operation for opening or closing a valve device of the gas cylinder that is to be used. Consequently, it is possible to omit a pneumoperitoneum device, which has been conventionally used to automatically regulate a flow rate of gas for pneumoperitoneum. Note that the operation for opening or closing the valve device of the gas cylinder is not necessarily performed by the person holding the optical viewing tube. The valve device may be provided to a main body of the gas cylinder, or may be provided separately from the cylinder.

The accommodation section of the rigid laparoscopic device may also serve as a grip member which can be gripped to hold the optical viewing tube.

In order to obtain an image of an observation object appropriately with the optical viewing tube, it is necessary to adjust the optical viewing tube at an appropriate position and an appropriate angle and to keep the adjusted state. By configuring the accommodation section so that the accommodation section serves as the grip member of the optical viewing tube, it is possible to hold the endoscope stably, while effectively utilizing the space of the accommodation section. For example, the "grip member" herein may be shaped like a gun grip of the optical viewing tube that is taken as a barrel of a gun, or may have another shape. In the case where the grip member is shaped like a gun grip, a user can operate the entire rigid laparoscopic device including the optical viewing tube as if to operate a gun (as a tool obtained as a result of the pursuit for operability). Consequently, such a configuration can provide better feeling of use and better operability.

In addition, the accommodation section may be provided with a valve operating section used to open and close a valve of the gas cylinder.

With such a configuration, the user can perform the operation for opening or closing the valve device, even in a case where the gas cylinder is accommodated in a form that does not allow the user to touch the main body of the gas cylinder, for example. This makes it possible to enhance freedom of selection of the accommodation form for the gas cylinder.

In addition, the accommodation section may be configured to allow a power source of the light source and/or the gas cylinder to be detachable from the accommodation section. Consequently, when the power source runs out of power, it is possible to replace only the power source. Meanwhile, when the gas cylinder runs out of gas, it is possible to replace only the gas cylinder. This is far more economical than replacement of the whole of the device.

In addition, the accommodation section may be detachably provided to the optical viewing tube.

With such a configuration, it is possible to replace the power source and the gas cylinder together with the accommodation section. This can reduce the burden of securing of cleanliness during a surgery.

In addition, the optical viewing tube of the endoscopic device may be integrally couplable with a converting device configured to convert an image obtained by the observation optical system into an electric signal.

With such a configuration, it is possible to transfer the electric signal to an image display device to cause the image display device to display an image of the observation object, thereby allowing two or more people to simultaneously observe the state of the operative field in a similar manner to the conventional systems.

In addition, the optical viewing tube may be integrally couplable further with a display device configured to display the image having been converted into the electric signal by the converting device.

The endoscopic system including the image display device as a separate component requires a space, wiring, and the like for the image display device. Meanwhile, the endoscopic system including the display device integrally couplable with the optical viewing tube can be made simple as a whole. Furthermore, with the configuration described above, the user can see the observation object with an unstrained natural posture, unlike a case where the user directly looks into the optical viewing tube.

Note that the image display device is not an essential component for the present invention. Alternatively, for example, an eyepiece may be attached to the optical viewing tube to allow the user to directly look into the optical viewing tube to observe the state inside the body cavity.

With such a configuration, it is possible to omit the video processor and the display device. This can significantly reduce the space for the devices used for a laparoscopic surgery, thereby making it possible to provide a rigid laparoscopic system that is simpler and lower in cost.

In addition, the rigid laparoscopic device according to the present invention may be configured such that the accommodation section is sterilizable.

The endoscopic device is assumed to be sterilized before the endoscopic device is packed after being manufactured as medical equipment. Also, endoscopic device is assumed to be sterilized for reuse in a medical site. In this regard, with the accommodation section that is sterilizable, it is possible to reduce the burden of securing cleanliness of the endoscope. Specifically, the accommodation section may be made of a material excellent in heat resistance and/or chemical resistance, and may be configured to have high airtightness, high water-tightness, and high pressure resistance.

In the rigid laparoscopic device, the accommodation section may be configured to be disposable. The "disposable" herein means not only the one that is to be disposed once it is used, but also a semidisposable one designed to be used several times to some tens of times.

In addition, the rigid laparoscopic device may be configured such that the gas cylinder has a body part whose surface is at least partially covered with a heat insulator. For example, during replacement of the gas cylinder that has run out of gas, a user may potentially be frostbitten if he/she directly touches the surface of the cylinder having been cooled by heat of evaporation. Furthermore, the gas cylinder having been cooled may negatively affect the peripheral elements. These risks can be reduced by the configuration including the heat insulator. The heat insulator may be directly provided on the surface of the cylinder, or may be provided to the cylinder accommodation part of the accommodation section.

### Advantageous Effects of Invention

According to the present invention, it is possible to provide a rigid laparoscopic system that is simpler and lower in cost; and a rigid laparoscopic device for such a rigid laparoscopic system. Furthermore, it is possible to further enhance the integrity or rigidity of the whole of the rigid laparoscopic system and the rigid laparoscopic device, and also to allow a greater number of functions of the system and the device to be collected into a form usable by a user with his/her single hand.

### Brief Description of Drawings

FIG. 1 is a view illustrating an example of a rigid laparoscopic device according to a first embodiment of the present invention that is in use.
FIG. 2 is a lateral cross-sectional view of the rigid laparoscopic device according to the first embodiment of the present invention.
FIG. 3 is an overall view of a rigid laparoscopic system according to a second embodiment of the present invention.
FIG. 4 is a view illustrating an accommodation section according to the second embodiment of the present invention.
FIG. 5 is a view illustrating a conventional system for a laparoscope.

### Description of Embodiments

The following describes specific embodiments of the present invention with reference to the drawings. Unless specifically stated otherwise, the dimensions, materials, shapes, relative positions, and the like of the components described in the embodiments described below are not intended to limit the technical scope of the invention to them.

Note that the expressions "base end" and "proximal end" herein mean a first end closer to a user, and the expressions "tip end" and "distal end" herein mean a second end opposite to the first end.

### <First embodiment>

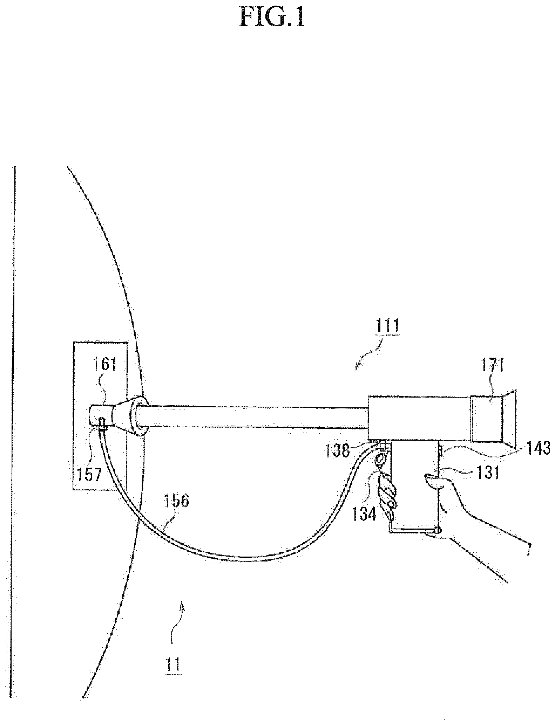

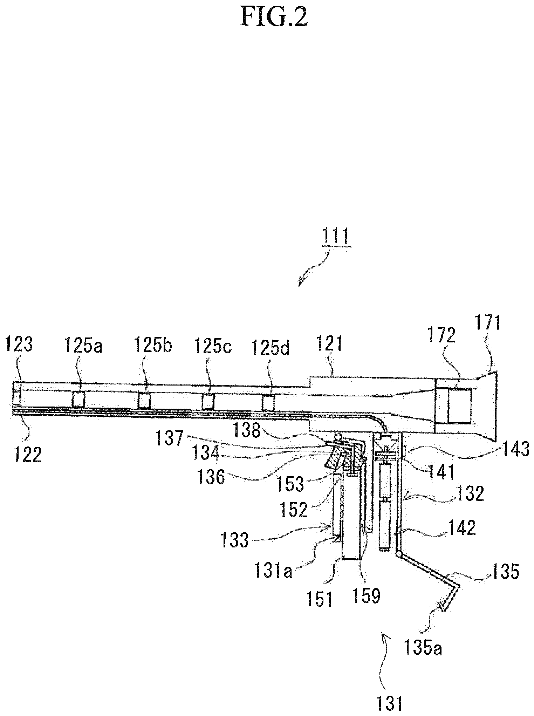

First, the following describes a first embodiment of the present invention. FIG. 1 is a view illustrating an example of an endoscopic device according to the first embodiment of the present invention that is in use. FIG. 1 illustrates a rigid laparoscopic device 111 as well as a trocar 161 provided with a gas port 157 and an air feeding tube 156. The rigid laparoscopic device 111, the trocar 161, and the air feeding tube 156 constitute a rigid laparoscopic system 11 for internal observation of the body of a patient. FIG. 2 is a lateral cross-sectional view of the rigid laparoscopic device 111 illustrated in FIG. 1.

As illustrated in FIGS. 1 and 2, the rigid laparoscopic device 111 includes a light guide cable 122 (e.g., an optical fiber cable) that is an illumination optical system, an objective lens 123 that is an observation optical system, an optical viewing tube 121 incorporating relay lenses 125a to 125d for transmitting an image, an eyepiece 171 connected (e.g., by a screw) to a base end of the optical viewing tube, and an accommodation section 131 including a light source device section 132 and a gas feeding section 133. Note that a length of the optical viewing tube 121 is not particularly limited, and may be any length as long as it allows the optical viewing tube 121 to be inserted into a small incision made on the abdominal wall and to observe the abdominal cavity and an organ in the abdominal cavity. The optical viewing tube 121 does not need to be as long as a soft gastrointestinal endoscope.

The eyepiece 171 is provided with an eye lens 172. A user looking into the eyepiece will see an image taken by the objective lens 123 at a tip end of the optical viewing tube and then transferred to the eyepiece by the relay lenses 125a to 125d.

In the present embodiment, the user looks into the eyepiece 171 to see an image captured at the tip end of the rigid laparoscope. Alternatively, the optical viewing tube 121 may be connected to a camera and a video processor, and an image captured by the optical viewing tube may be displayed on a separately-provided display device.

Next, the following describes details of the accommodation section 131. The accommodation section 131 is shaped like a gun grip shape to serve also as a grip member of the rigid laparoscopic device 111. The accommodation section 131 has a bottom made of a lid member 135 that is openable. The lid member 135 is pivotally supported at a lower end of the light source device section 132 such that the lid member 135 is rotatable. When the lid member 135 comes into a closed state, a claw 135a is engaged with a groove 131a, so that the lid member 135 is fastened.

The accommodation section 131 of the present embodiment is integrated with the optical viewing tube 121. Alternatively, the accommodation section 131 may be configured to be detachable via male and female fasteners, for example. The accommodation section 131 and the optical viewing tube 121 of the present embodiment are firmly fastened to each other while the accommodation section 131 and the optical viewing tube 121 are in a coupled state, regardless of whether the accommodation section 131 and the optical viewing tube 121 are configured to be detachable. This makes it possible to maintain rigidity of the entire device. A way of coupling between the accommodation section 131 and the optical viewing tube 121 is not limited to those described above. Alternatively, the accommodation section 131 and the optical viewing tube 121 may be coupled by a screw, an adhesive, welding, or the like. Further alternatively, the coupling may be performed with a male screw fixed to one of the accommodation section 131 and the optical viewing tube 121 and a female screw provided to the other. One of the male and female screws may be screwed into the other.

The inside of the accommodation section 131 is divided into the light source device section 132 for supplying light to the light guide cable 122 and a pneumoperitoneum gas feeding section 133 for supplying gas to the air feeding tube 156. The light source device section 132 is provided with a battery accommodation section 142, and the pneumoperitoneum gas feeding section 133 is provided with a gas cylinder accommodation section 159.

When the lid member 135 is opened as illustrated in FIG. 2, a battery and a gas cylinder can be attached to or detached from the battery accommodation section 142 and the gas cylinder accommodation section 159, respectively, through the bottom of the accommodation section 131.

The light source device section 132 of the present embodiment is configured such that only the battery is detachable from the light source device section 132. Alternatively, the light source device section 132 may be configured such that the battery as well as a light source 141 are detachable at a time.

When a light source switch 143 is turned ON, the light source 141 (e.g., an LED) emits light powered by the battery. The light emitted from the light source 141 passes through the light guide cable 122, and is then emitted from the tip end of the optical viewing tube 121.

The specification of the battery to be used is not particularly limited. In addition, the battery to be used may be either a primary battery or a secondary battery. In the present embodiment, the light source switch 143 is provided to switch between ON and OFF of the illumination. Alternatively, the light source switch 143 may be omitted, and the illumination may be configured to be turned ON continuously after the battery is set.

Next, the following describes details of the pneumoperitoneum gas feeding section 133 and pneumoperitoneum means of the present embodiment. A gas cylinder 151 used in the present embodiment is a small cylinder (e.g., 10 cm in height and 3 cm in diameter) which has a main body with a relatively small content (e.g., 25 g) of carbon dioxide gas and which incorporates a valve device 152. The main body of the gas cylinder 151 has an upper portion provided with a stem 153 that operates interlockingly with the valve device 152.

When the gas cylinder 151 runs out of the gas filled therein, the gas cylinder 151 can be replaced with a new one even during a surgery. Thus, the content and size of the gas cylinder are not particularly limited to those described above, as long as they allow the gas cylinder to be accommodated in the gas cylinder accommodation section 159.

A heat insulator may be provided on a surface of the gas cylinder 151. For example, a foamed rubber covers at least partially a body part of the gas cylinder 151. Alternatively, the heat insulator may be formed inside the gas cylinder accommodation section 159, rather than on the surface of the gas cylinder 151. This can prevent the surface of the cylinder having been cooled by heat of evaporation from being touched. Also, this can reduce the risk that the gas cylinder having been cooled may negatively affect the peripheral elements.

Above the pneumoperitoneum gas feeding section 133, a trigger-lever type valve operating section 134 is provided. The valve operating section 134 includes a stem engagement part 136 configured to be engaged with the stem 153 of the gas cylinder 151 and a gas flow passage 137 extending from the stem engagement part 136 to a nozzle 138. The gas cylinder 151 is accommodated in the gas cylinder accommodation section 159 such that the stem 153 is fitted into the stem engagement part 136 of the valve operating section 134.

Operating the valve operating section 134 in this state as if to pull the trigger pushes down the stem engagement part 136. This opens the valve device 152 of the gas cylinder 151, thereby causing the gas to be discharged from the nozzle 138 of the valve operating section 134. The gas discharged from the nozzle reaches the gas port 157 of the trocar 161 through the air feeding tube 156, and is then fed into the abdominal cavity of the patient.

The present embodiment does not include a down regulator for automatically regulating a pressure, although the down regulator is included in the pneumoperitoneum device of the conventional laparoscopic system. As a premise, in the present embodiment, a gas pressure is regulated by manually operating the valve operating section 134. This is based on a finding that pneumoperitoneum can be performed in an adequately suitable manner by manually regulating a gas pressure while directly observing the inside of the abdominal cavity. In the present embodiment, the endoscopic device 111 has a rigid integrated structure that enables an operator to operate the laparoscopic system 11 with his/her single hand. Consequently, a single operator can perform the pneumoperitoneum work and another work at the same time. Thus, it is far easier to perform the pneumoperitoneum work while manually regulating a gas pressure.

The optical viewing tube 121 and the accommodation section 131 may be configured to be sterilizable. Specifically, the optical viewing tube 121 and the accommodation section 131 may be made of a metal (e.g., a stainless steel) or a heat-resistant resin (e.g., polypropylene) to achieve high airtightness and high water-tightness so that the optical viewing tube 121 and the accommodation section 131 can be subjected to sterilization by high-pressure steam in an autoclave. Also, the optical viewing tube 121 and the accommodation section 131 may be made of a chemical-resistant resin (e.g., fluororesin) so that the optical viewing tube 121 and the accommodation section 131 can be subjected to chemical sterilization.

Further alternatively, the rigid laparoscopic device 111 may be configured to be disposable. In this case, the gas cylinder and/or the battery may not be replaceable, and thus the accommodation section 131 may not be openable via the lid member 135, for example. In addition, the rigid laparoscopic device 111 may be made of an inexpensive resin (e.g., polyethylene), and may not necessarily have high airtightness or waterproofing property. Thus, it is possible to provide the rigid laparoscopic device 111 at a quite low cost. In this case, the whole of the rigid laparoscopic device 111 may be configured to be disposable. Alternatively, part of the rigid laparoscopic device 111, for example, only the accommodation section 131 may be configured to be disposable. For example, the accommodation section 131 configured to be disposable may be partially opened such that the gas cylinder and the power source arranged inside the accommodation section 131 are partially exposed. By further simplifying the structure of the accommodation section 131 in this manner, the accommodation section 131 can be made easily disposable.

As described above, in the present embodiment, the optical viewing tube 121 having a higher rigidity and a shorter length than a gastrointestinal endoscope is firmly combined with the accommodation section 131. This allows an operator to make use of the illumination function, the observation function, and the pneumoperitoneum function with his/her single hand. Furthermore, the rigid laparoscopic system 11 according to the present embodiment does not need an external power source. This eliminates the disadvantage that a range of use of the rigid laparoscopic system 11 is limited by a power-supply line and the disadvantage that the rigid laparoscopic system 11 is pulled by the power-supply line while the rigid laparoscopic system 11 is in use. Consequently, freedom of use is significantly enhanced. Moreover, the rigid laparoscopic system 11 according to the present embodiment is smaller in size, lighter in weight, and lower in cost, and does not need a power supply. Thus, the rigid laparoscopic system 11 according to the present embodiment is easy to use even in disaster sites and developing countries.

Here, in a case where the accommodation section 131 is simply provided to a soft gastrointestinal endoscope, such a soft gastrointestinal endoscope requires, e.g., a dial for operating the endoscope, in addition to the accommodation section 131. Furthermore, since the gastrointestinal endoscope is typically far longer and softer than the rigid laparoscope, a user cannot use the gastrointestinal endoscope with his/her single hand. Hence, the rigid laparoscopic device 111 and the rigid laparoscopic system 11 of the present embodiment bring about greater advantages, as compared to the configuration in which the accommodation section 131 is simply provided to the soft gastrointestinal endoscope.

In addition, the rigid laparoscopic device 111 and the rigid laparoscopic system 11 of the present embodiment can employ a smartphone and a tablet computer as the video processor and the monitor. Consequently, it is possible to enhance the functions of the device, without impairing the compact structure and usability of the device.

### <Second embodiment>

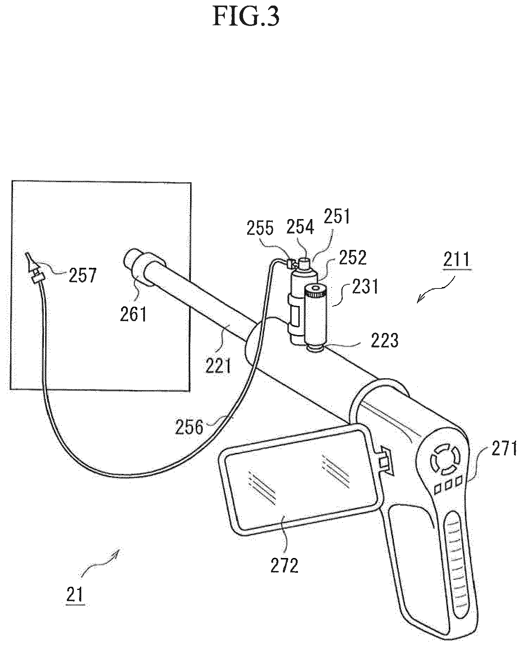

The following describes a second embodiment of the present invention. FIG. 3 is a view illustrating a rigid laparoscopic system 21 including a rigid laparoscopic device 211 according to the present embodiment. In FIG. 3, the rigid laparoscopic device 211, a pneumoperitoneum needle 257, and an air feeding tube 256 are illustrated. As illustrated in FIG. 3, the rigid laparoscopic device 211 includes an optical viewing tube 221, an accommodation section 231, a gas cylinder 251 supported by the accommodation section 231, and a handy video camera 271 provided with a liquid crystal monitor and connected to a base end of the optical viewing tube 221. In addition, in a similar manner to the first embodiment, the optical viewing tube 221 incorporates a light guide cable (not illustrated) of an illumination optical system and a lens (not illustrated) of an observation optical system.

The handy video camera 271 may be a simple one provided only with an image display function and a video recording function. Alternatively, the handy video camera 271 may be, e.g., a commercially available video camera having other functions such as a zoom function and/or a brightness correction function.

Note that the handy video camera 271 is not an essential component for the present invention. The rigid laparoscopic device 211 may be integrally provided only with an image capturing device (camera), and may be configured to cause an image captured by the image capturing device to be displayed on a separately-provided image display device (monitor) so that the image can be observed by two or more people simultaneously. Furthermore, the image captured by the image capturing device may be recorded in a separately-provided recording device.

Moreover, instead of the handy video camera 271, a portable information terminal such as a mobile phone or a tablet terminal may be connected to enable observation of an image by use of a camera function of the portable information terminal and recording of the image in a recording medium of the portable information terminal. Note that each of the portable information terminal, the handy video camera 271, and the image capturing device described above corresponds to a "converting device" recited in the claims of the present application.

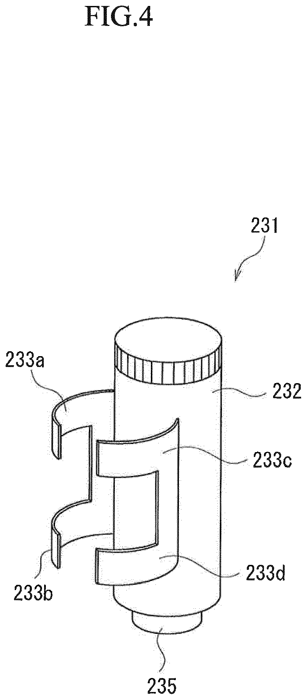

Next, the following describes the accommodation section 231 with reference to FIG. 4. As illustrated in FIG. 4, the accommodation section 231 includes a light source device accommodation section 232 and a gas cylinder holding section constituted by four arcuate members 233a to 233d extending from the light source device accommodation section 232. The accommodation section 231 having such a configuration can detachably hold the gas cylinder 251 by the members 233a to 233d.

In addition, the light source device accommodation section 232 has an accommodation-section-side connector 235 for connection with the optical viewing tube 221. The accommodation-section-side connector 235 is detachably connected to a main-body-side connector 223, which is provided to the optical viewing tube 221. Means for the connection is not particularly limited. For example, the connection may be achieved by engagement between screws, i.e., a male screw that serves as the accommodation-section-side connector 235 and a female screw that serves as the main-body-side connector 223.

At the main-body-side connector 223, one end of the light guide cable (not illustrated) incorporated in the optical viewing tube 221 is positioned. Light emitted from a light source (not illustrated) passes through the light guide cable, and is then emitted toward a tip end of the optical viewing tube.

Next, the following describes pneumoperitoneum means of the present embodiment. The gas cylinder 251 used in the present embodiment is a small cylinder which has a main body with a relatively small content of carbon dioxide gas and which incorporates a valve. Here, the gas cylinder 251 has a body part provided with a heat insulator 252. The heat insulator 252 is made of a foamed rubber covering the body part of the gas cylinder 251. This can prevent the risk that a user may touch the gas cylinder 251 having been cooled by heat of evaporation and be frostbitten.

An upper portion of a main body of the gas cylinder 251 has a push button 254 which is configured to operate interlockingly with a valve device in the cylinder and which is provided with a nozzle 255. Pushing down the push button 254 opens the valve device incorporated in the main body, thereby allowing carbon dioxide gas filled in the cylinder to be fed into the abdominal cavity of the patient through the nozzle 255, the air feeding tube 256, and the pneumoperitoneum needle 257.

The present invention is not limited to the configurations of the embodiments described above. The present invention also encompasses configurations of other rigid laparoscopic devices in which an optical viewing tube and a accommodation section storing a light source device and a pneumoperitoneum device are integrally arranged.

The rigid laparoscopic system according to the present invention is not necessarily limited to laparoscopic surgeries for human. That is, with the rigid laparoscopic system according to the present invention, an operator can move rapidly even while holding the rigid laparoscopic system, and therefore the rigid laparoscopic system according to the present invention is applicable also to treatments for animals that move unpredictably. Hence, the rigid laparoscopic system according to the present invention is applicable to laparoscopic surgeries for pet animals and domestic animals, such as dogs and cats.

### Reference Signs List

- 111, 211: rigid laparoscopic device
- 121, 221: optical viewing tube
- 131, 231: accommodation section
- 134: valve operating section
- 141: light source
- 151, 251: gas cylinder
- 156, 256: air feeding tube
- 157: gas port of trocar
- 161, 261: trocar
- 171: eyepiece
- 257: pneumoperitoneum needle
- 271: handy video camera

## Claims

1. A rigid laparoscopic device comprising:
an optical viewing tube including an observation optical system and an illumination optical system,
wherein
an accommodation section in which a light source for supplying illumination light to the illumination optical system and a gas cylinder for supplying gas for pneumoperitoneum are accommodated is provided to the optical viewing tube to allow the optical viewing tube, the light source, and the gas cylinder to be held integrally and with rigidity.

2. The rigid laparoscopic device according to claim 1, wherein the accommodation section is a grip member which can be gripped to hold the optical viewing tube.

3. The rigid laparoscopic device according to claim 1 or 2, wherein the accommodation section is provided with a valve operating section used to open and close a valve of the gas cylinder.

4. The rigid laparoscopic device according to any one of claims 1 to 3, wherein the accommodation section is configured to allow a power source of the light source and/or the gas cylinder to be detachable from the accommodation section.

5. The rigid laparoscopic device according to any one of claims 1 to 4, wherein the accommodation section is detachably provided to the optical viewing tube.

6. The rigid laparoscopic device according to any one of claims 1 to 5, wherein the optical viewing tube is integrally couplable with a converting device configured to convert an image obtained by the observation optical system into an electric signal.

7. The rigid laparoscopic device according to claim 6, wherein the optical viewing tube is integrally couplable further with a display device configured to display the image having been converted into the electric signal by the converting device.

8. The rigid laparoscopic device according to any one of claims 1 to 7, wherein the accommodation section is sterilizable.

9. The rigid laparoscopic device according to any one of claims 1 to 8, wherein the accommodation section is configured to be disposable.

10. The rigid laparoscopic device according to any one of claims 1 to 9, wherein the gas cylinder has a body part whose surface is at least partially covered with a heat insulator.
